# EUROPEAN PATENT SPECIFICATION

(11) **EP 1 919 530 B1**
(45) Date of publication and mention of the grant of the patent: **09.11.2011**
(21) Application number: 06780252.0
(22) Date of filing: 31.07.2006
(51) Int. Cl.: A61L 24/06

(54) **BONE CEMENT AND METHODS OF USE THEREOF**
KNOCHENZEMENT UND ANWENDUNGSVERFAHREN DAFÜR
CIMENT OSSEUX ET PROCEDES D'UTILISATION DUDIT CIMENT

(30) Priority: 31.07.2005 WO PCT/IL2005/000812; 22.02.2006 WO PCT/IL2006/000239; 22.02.2006 US 360251; 16.03.2006 IL 17434706; 25.10.2005 US 729505 P; 22.11.2005 US 738556 P; 26.01.2006 US 762789 P; 02.02.2006 US 765484 P
(43) Date of publication of application: 14.05.2008
(73) Proprietor: Depuy Spine, Inc., Raynham, MA 02767 (US)
(72) Inventor: BEYAR, Mordechay, 38900 Caesarea (IL); GLOBERMAN, Oren, 46910 Kfar-Shmaryahu (IL)
(74) Representative: Orr, Robert
(86) International application number: PCT/IB2006/052612
(87) International publication number: WO 2007/015202

(56) References cited:
- EP-A1- 0 177 781
- WO-A-2004/019810
- WO-A-2004/071543
- WO-A1-99/18894
- FR-A1- 2 638 972
- LEWIS GLADIUS: "Properties of acrylic bone cement: State of the art review" JOURNAL OF BIOMEDICAL MATERIALS RESEARCH, vol. 38, no. 2, 1997, pages 155-182, XP002432739 ISSN: 0021-9304
- LEWIS GLADIUS: "Toward standardization of methods of determination of fracture properties of acrylic bone cement and statistical analysis of test results" JOURNAL OF BIOMEDICAL MATERIALS RESEARCH, vol. 53, no. 6, 2000, pages 748-768, XP002432740 ISSN: 0021-9304

## Description

### FIELD OF THE INVENTION

The present invention relates to bone cement materials.

### BACKGROUND TO THE INVENTION

It is common to employ cement to repair bones in a variety of clinical scenarios.

For example, compression fractures of the vertebrae, which are a common occurrence in older persons, cause pain and/or a shortening (or other distortion) of stature. In a procedure known as vertebroplasty cement is injected into a fractured vertebra. Vertebroplasty stabilizes the fracture and reduces pain, although it does not restore the vertebra and person to their original height. In vertebroplasty the cement is typically injected in a liquid phase so that resistance to injection is not too great. Liquid cement may unintentionally be injected outside of the vertebra and/or may migrate out through cracks in the vertebra.

In another procedure, known as kyphoplasty, the fracture is reduced by expanding a device, such as a balloon inside the vertebra and then injecting a fixing material and/or an implant. Kyphoplasty reduces the problem of cement leakage by permitting a lower pressure to be used for injection of the cement.

In general, polymeric cements become more viscous as the polymer chain grows by reacting directly with the double bond of a monomer. Polymerization begins by the "addition mechanism" in which a monomer becomes unstable by reacting with an initiator, a volatile molecule that is most commonly a radical (molecules that contain a single unpaired electron). Radicals bond with monomers, forming monomer radicals that can attack the double bond of the next monomer to propagate the polymer chain. Because radicals are so transient, initiators are often added in the form of an un-reactive peroxide form which is stable in solution. Radicals are formed when heat or light cleaves the peroxide molecule. For applications in which high temperatures are not practical (such as the use of bone cement in vivo), peroxide is typically cleaved by adding a chemical activator such as N, N-dimethyl-p-toluidine. (Nussbaum DA et al: "The Chemistry of Acrylic Bone Cement and Implication for Clinical Use in Image-guided Therapy", J Vase Interv Radiol (2004); 15:121-126).

Examples of commercially available viscous bone cements include, but are not limited to, CMW® Nos. 1, 2 and 3 (DePuy Orthopaedics Inc.; Warsaw, IN, USA) and Simplex(TM) -P and -RO (Stryker Orthopaedics; Mahwah, NJ, USA). These cements are characterized by a liquid phase after mixing and prior to achieving a viscosity of 500 Pascal-second. In a typical use scenario, these previously available cements are poured, while in a liquid phase, into a delivery device.

There have also been attempts to reduce cement leakage by injecting more viscous cement, for example, during the doughing time and the beginning of polymerization. However, the viscous materials, such as hardening PMMA, typically harden very quickly once they reach a high viscosity. This has generally prevented injection of viscous materials in orthopedic procedures.

Some bone fixing materials, such as polymethylmethacrylate (PMMA), emit heat and possibly toxic materials while setting.

US patents and publication 4,969,888, 5,108,404, 6,383,188, 2003/0109883, 2002/0068974, 6,348,055, 6,383,190, 4,494,535, 4,653,489 and 4,653,487 describe various tools and methods for treating bone.

US patent publication 2004/0260303 teaches an apparatus for delivering bone cement into a vertebra.

Pascual, B., et al., "New Aspects of the Effect of Size and Size Distribution on the Setting Parameters and Mechanical Properties of Acrylic Bone Cements," Biomaterials, 17(5): 509-516 (1996) considers the effect of PMMA bead size on setting parameters of cement.

Hernandez, et al., (2005) "Influence of Powder Particle Size Distribution on Complex Viscosity and Other Properties of Acrylic Bone Cement for Vertebroplasty and Kyphoplasty" Wiley International Science D01:10:1002/jbm.b.30409 (pages 98-103) considers the effect of PMMA bead size distribution on setting parameters of cement. Hernandez suggests that it is advantageous to formulate cement with a liquid phase to facilitate injection.

US 5,276,070 to Arroyo discloses use of acrylic polymers with a molecular weight in the range of 0.5 to 1.5 million Daltons in formulation of bone cement.

US 5,336,699 to Cooke discloses use of acrylic polymers with a molecular weight of about one hundred thousand Daltons in formulation of bone cement.

The paper "Properties of Acrylic Bone Cement: State of the Art Review" by Lewis (Journal of Biomedical Materials Research, Vol 38, 1997, pages 155-182) discloses details of commercially available acrylate polymer bone cement materials, including details of the viscosity of the materials during mixing of liquid and powder components. It is stated that the ideal cement would display a low value of the dynamic viscosity and practical invariance of the viscosity with dime during the working period (typically 3 to 6 minutes after the start of mixing), followed by a very short time to full polymerisation.

The paper "Toward Standardisation of Methods of Determination of Fracture Properties of Acrylic Bone Cement and Statistical Analysis of Test Results" by Lewis and Nyman (Journal of Biomedical Materials Research, Vol 53, 2000, pages 748-768) is a review of methods for measuring the fatigue and related performance characteristics of acrylate polymer bone cement materials.

WO-A-2004/019810 discloses a bone cement replacement material which comprises barium sulphate, 65 µm beads of a PMMA-styrene copolymer, and 750 µm PMMA beads. This powder component is mixed with a liquid comprising methyl methacrylate monomer, dimethyl-p-toluidine and hydroquinone. The mixture reaches its dough state at three minutes after mixing. It sets to its final polymerised state at nine minutes after mixing.

WO-A-99/18894 discloses a PMMA based implantable composition which is made from a slurry of PMMA, barium sulphate powder, and a methacrylate-styrene copolymer, with added particles of barium sulphate and optionally tungsten. The powder is mixed with a liquid phase containing methacrylate monomer, N,N-dimethyl-p-toluidine and hydroquinone.

FR-2638972 discloses a bone cement material formed by mixing a solid component containing methyl methacrylate polymer, an organic peroxide such as benzoyl peroxide, and zirconium dioxide with a liquid component containing methyl methacrylate, N-butyl methacrylate and N,N-dimethyl-p-toluidine.

WO-A-2004/071543 discloses a substitute bone material which is formed by mixing a solid component comprising PMMA and benzoyl peroxide with a liquid component comprising methyl methacrylate monomer and N,N-dimethyl-p-toluidine. The material also includes an organic X-ray contrast agent such as iopromidium in an aqueous solution of hyaluronic acid. The viscosity of the mixture can be in the range 200,000 to 300,000 centipoise after 4 minutes have elapsed following mixing of the components.

EP-A-177781 discloses a process for preparing a bone cement material in which beads of an acrylate polymer or a methacrylate polymer are broken down by mechanical and/or chemical treatment so as to increase the surface area of the beads. The treated beads are then reacted with acrylic acid and/or methacrylic acid derivatives and a polymerisation catalyst.

### SUMMARY OF THE INVENTION

The invention provides a bone cement as defined in claim 1.

The bone cement of the invention undergoes a rapid transition from separate liquid monomer and powdered polymer components to a single phase characterized by a high viscosity when the components are mixed together with substantially no intervening liquid phase.

Mixing is deemed complete when 95-100% of the polymer beads are wetted by monomer. In an exemplary embodiment of the invention, mixing is complete in within 60, optionally within 45, optionally within 30 seconds.

In an exemplary embodiment of the invention, the cement is characterized by a working window of several minutes during which the viscosity remains high prior to hardening of the cement. Optionally, viscosity during the working window does not vary to a degree which significantly influences injection parameters. In an exemplary embodiment of the invention, viscosity increases by less than 10% during a sub-window of at least 2 minutes during the working window. Optionally, the viscosity in the working window does not exceed 500, optionally 1,000, optionally 1,500, optionally 2,000 Pascal-second or lesser or greater or intermediate values. In an exemplary embodiment of the invention, the working window lasts 6, optionally 8, optionally 10, optionally 15 minutes or lesser or greater or intermediate times. Optionally, ambient temperature influences a duration of the working window. In an exemplary embodiment of the invention, the cement can be cooled or heated to influence a length of the working window.

An aspect of some embodiments of the invention relates to formulations of bone cement which rely upon three or more, sub-populations of polymer beads which are mixed with liquid monomer.

According to exemplary embodiments of the invention, sub-populations may be characterized by physical size and/or geometry, and/or density. In an exemplary embodiment of the invention, size based and MW based sub-populations are defined independently. In an exemplary embodiment of the invention, the sub-populations are selected to produce desired viscosity characterization and/or polymerization kinetics. The polymer beads comprise polymethylmethacrylate (PMMA) and/or a PMMA or MMA styrene copolymer. Optionally, PMMA is employed in conjunction with a methylmethacrylate (MMA) monomer.

A high molecular weight sub-population contributes to a rapid transition to a high viscosity with substantially no liquid phase. A low molecular weight subpopulation contributes to a longer working window.

Optionally, a sub-population with small size contributes to rapid wetting of polymer beads with monomer solution. In an exemplary embodiment of the invention, rapid wetting contributes to a direct transition to a viscous cement with substantially no liquid phase.

In some cases a small percentage of beads may not belong to any relevant sub-population. The small percentage may be, for example 1%, 1.5%, 2%, 3%, 4%, 5% or lesser or intermediate or greater percentages.

In one exemplary embodiment of the invention, a first sub-population comprising 95 to 97% (w/w) of the total PMMA beads can be characterized by an average MW of 270,000-300,000 Dalton; a second sub-population (2-3% w/w) can be characterized by an average MW of 3,500,000-4,000,000 Dalton; and a third sub-population (0-3% w/w) can be characterized by an average MW of 10,000-15,000 Dalton.

In an exemplary embodiment of the invention, the polymer beads are characterized by a high surface area per unit weight. Optionally, the beads have a surface area of 0.5 to 1, optionally 0.5 to 0.8 optionally about 0.66 m⁻²/gram or intermediate or lesser or greater values. Optionally, the high surface area/weight ratio improves wetting properties and/or shortens polymerization times, for example by contributing to polymer monomer contact.

In an exemplary embodiment of the invention, a cement characterized by an immediate transition to high viscosity is injected during a working window in a vertebroplasty or kyphoplasty procedure. Optionally, injection is under sufficient pressure to move fractured bone, such as vertebral plates of a collapsed vertebra. Optionally, injection of viscous cement under high pressure contributes to fracture reduction and/or restoration of vertebral height.

In an exemplary embodiment of the invention, the material (e.g., bone cement) includes processed bone (from human or animals origin) and/or synthetic bone. Optionally, the cement has osteoconductive and/or osteoinductive behaviour. Additional additives as commonly used in bone cement preparation may optionally be added. These additives include, but are not limited to, barium sulfate and benzoyl peroxide.

According to some embodiments of the invention, a working window length is determined by an interaction between an immediate effect and a late effect. In an exemplary embodiment of the invention, the immediate effect includes MMA solvation and/or encapsulation of PMMA polymer beads. The immediate effect contributes to a high viscosity of the initial mixture resulting from solvation and/or friction between the beads. The late effect is increasing average polymer MW as the beads dissolve and the polymerization reaction proceeds. This increasing average polymer MW keeps viscosity high throughout the working window.

In an exemplary embodiment of the invention, a set of viscosity parameters are used to adjust a cement formulation to produce a cement characterized by a desired working window at a desired viscosity.

Optionally, the viscosity of the mixture remains between 500 and 2000 Pascal-second for a working window of at least 5 minutes after the initial period.

Optionally, the working window is at least 8 minutes long.

Optionally, the mixture includes Barium Sulfate.

Optionally, the PMMA is provided as a PMMA/styrene copolymer.

Optionally, the PMMA is provided as a population of beads divided into at least two sub-populations, each sub-population characterized by an average molecular weight.

Optionally, a largest sub-population of PMMA beads is characterized by an MW of 150,000 Dalton to 300,000 Dalton.

Optionally, a largest sub-population of PMMA beads includes 90-98% (w/w) of the beads.

Optionally, a high molecular weight sub-population of PMMA beads is characterized by an average MW of at least 3,000,000 Dalton.

Optionally, a high molecular weight sub-population of PMMA beads includes 2 to 3% (w/w) of the beads.

Optionally, a low molecular weight sub-population of PMMA beads is characterized by an average MW of less than 15,000 Dalton.

Optionally, a low molecular weight sub-population of PMMA beads includes 0.75 to 1.5 % (w/w) of the beads.

Optionally, the PMMA is provided as a population of beads divided into at least two sub-populations, each sub-population characterized by an average bead diameter.

Optionally, at least one bead sub-population characterized by an average diameter is further divided into at least two sub-sub-populations, each sub-sub-population characterized by an average molecular weight.

Optionally, the PMMA is provided as a population of beads divided into at least three sub-populations, each sub-population characterized by an average bead diameter.

Optionally, the cement further includes processed bone and/or synthetic bone.

Optionally, the cement is characterized in that the cement achieves a viscosity of at least 500 Pascal-second when 100% of a polymer component is wetted by a monomer component.

Optionally, the viscosity is at least 800 Pascal-second.

Optionally, the viscosity is at least 1500 Pascal-second.

Optionally, the viscosity is achieved within 2 minutes.

Optionally, the viscosity is achieved within 1 minute.

Optionally, the viscosity is achieved within 45 seconds.

In an exemplary embodiment of the invention, there is provided a bone cement comprising:
a polymer component; and
a monomer component,
wherein, contacting the polymer component and the monomer component produces a mixture which attains a viscosity greater than 200 Pascal-second within 1 minute from onset of mixing and remains below 2000 Pascal-second until at least 6 minutes from onset of mixing.

Optionally, the polymer component comprises an acrylic polymer.

In an exemplary embodiment of the invention, there is provided a particulate mixture formulated for preparation of a bone cement, the mixture comprising:
(a) 60 to 80% polymer beads comprising a main sub-population characterized by an MW of 150,000 Dalton to 300,000 Dalton and a high molecular weight sub-population characterized by an MW of 3,000,000 Dalton to 4,000,000 Dalton; and
(b) 20 to 40% of a material which is non-transparent with respect to X-ray.

Optionally, the polymer beads comprise a third subpopulation characterized by an MW of 10,000 Dalton to 15,000 Dalton.

In this disclosure there is also provided a method of making a polymeric bone cement, the method comprising:
(a) defining a viscosity profile including a rapid transition to a working window characterized by a high viscosity;
(b) selecting a polymer component and a monomer component to produce a cement confirming to the viscosity profile; and
(c) mixing the polymer component and a monomer component to produce a cement which conforms to the viscosity profile.

### BRIEF DESCRIPTION OF THE FIGURES

Exemplary non-limiting embodiments of the invention will be described with reference to the following description of embodiments in conjunction with the figures: Identical structures, elements or parts which appear in more than one figure are generally labeled with a same or similar number in all the figures in which they appear, in which:
Fig. 1 is a flow diagram illustrating an exemplary method 100 of preparation and behaviour of exemplary cements according to the present invention;
Fig. 2 is a graph of viscosity profiles depicting viscosity (Pascal-second) as a function of time (minutes) for an exemplary cement according to the invention and an exemplary prior art cement; and
Figs. 3 and 4 are graphs indicating viscosity as Newtons of applied force per unit displacement (mm) under defined conditions for exemplary cements according to the invention and illustrate the time window for injection which is both early and long.

### DETAILED DESCRIPTION OF EXEMPLARY EMBODIMENTS

Overview of preparation of exemplary bone cement

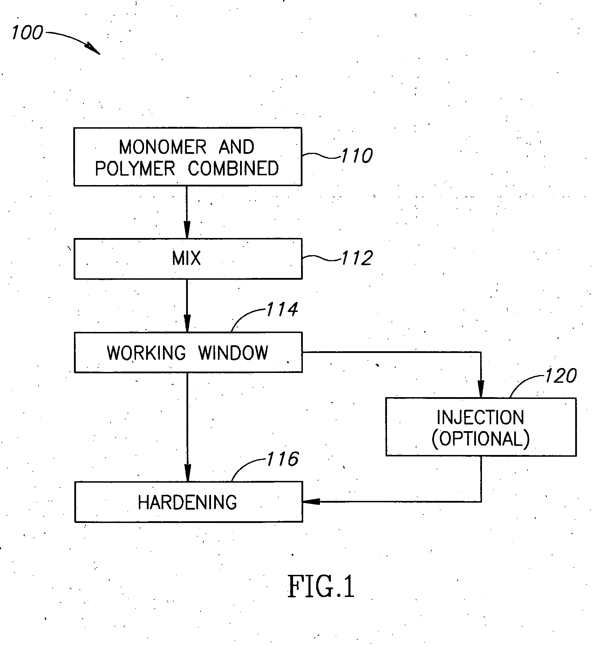

Fig. 1 is a flow diagram illustrating preparation and behavior of exemplary cements according to some embodiments of the present invention.

In an exemplary embodiment of the invention, a liquid monomer and a powdered polymer component of a bone cement are combined 110. Optionally, liquid monomer is poured onto powdered polymer.

According to various embodiments of the invention, average polymer molecular weight and/or polymer molecular weight distribution and/or polymer bead size is precisely controlled in order to influence polymerization kinetics and/or cement viscosity. Alternatively or additionally, polymer and/or monomer components may contain ingredients which are not directly involved in the polymerization reaction.

In an exemplary embodiment of the disclosure, the average molecular weight of the acrylic polymer in all the beads is in the range of about 300,000 to 400,000, optionally about 373,000 Dalton. This average MW for all beads was determined experimentally for a batch of beads which produced cement with a desired polymerization profile.

Optionally, the polymer beads are provided as part of an acrylic polymer mixture, for example a mixture including barium sulfate. At 112 the components are mixed until the polymer is wetted by the monomer. Optionally, when wetting is 95 to 100% complete, the mixture has achieved a desired high viscosity, for example 500 Pascal-second or more. Optionally, mixing 112 is complete within 1, 5, 10, 15, 30, 60, 90, 120 or 180 seconds. In a modem medical facility, it can be advantageous to shorten the mixing time in order to reduce the demand on physical facilities and/or medical personnel. A saving of even 1 to 2 minutes with respect to previously available alternatives can be significant. In an exemplary embodiment of the invention, mixing 112 is conducted in a mixing apparatus of the type disclosed in US-A-2008/212405.

After mixing 112 is complete, a working window 114 during which the cement remains viscous but has not fully hardened occurs. According to various exemplary embodiments of the invention, working window 114 may be about 2, 5, 8, 10, 15 or 20 minutes or intermediate or greater times. The duration of the working window may vary with the exact cement formulation and/or ambient conditions (e.g. temperature and/or humidity). Formulation considerations include, but are not limited to polymer MW (average and/or distribution), polymer bead size, concentrations of non-polymerizing ingredient and polymer: monomer ratio.

Working window 114, permits a medical practitioner sufficient time to load a high pressure injection device and inject 120 the cement into a desired location. Optionally, an injection needle or cannula is inserted into the body prior to, or concurrent with mixing 112 so that window 114 need only be long enough for loading and injection 120. Exemplary injection systems are disclosed in US-A-2007/027230.

In an exemplary embodiment of the invention, hardening 116 to a hardened condition occurs after working window 114. The cement hardens 116 even if it has not been injected.

### Advantages with Respect to Relevant Medical Procedures

In an exemplary embodiment of the invention, cement with a viscosity profile as described above is useful in vertebral repair, for example in vertebroplasty and/or kyphoplasty procedures.

Optionally, use of cement which is viscous at the time of injection reduces the risk of material leakage and/or infiltrates into the intravertebral cancellous bone (interdigitaion) and/or reduces the fracture [see G Baroud et al, Injection biomechanics of bone cements used in vertebroplasty, Bio-Medical Materials and Engineering 00 (2004) 1-18]. Reduced leakage optionally contributes to increased likelihood of a positive clinical outcome.

In an exemplary embodiment of the invention, the viscosity of the bone cement is 500, optionally 1,000, optionally 1,500, optionally 2,000 Pascal-second or lesser or greater or intermediate values at the time injection begins, optionally 3, 2 or 1 minutes or lesser or intermediate times after mixing 112 begins. Optionally, the viscosity does not exceed 2,000 Pascal-second during working window 114. In an exemplary embodiment of the invention, this viscosity is achieved substantially as soon as 95-100% of the polymer beads are wetted by monomer.

Cement characterized by a high viscosity as described above may optionally be manually manipulated.

In an exemplary embodiment of the invention, cement is sufficiently viscous to move surrounding tissue as it is injected. Optionally, moving of the surrounding tissue contributes to fracture reduction and/or restoration of vertebral height.

An injected volume of cement may vary, depending upon the type and/or number of orthopedic procedures being performed. The volume injected may be, for example, 2-5 cc for a typical vertebral repair and as high as 8-12 cc or higher for repairs of other types of bones. Other volumes may be appropriate, depending for example, on the volume of space and the desired effect of the injection. In some cases, a large volume of viscous cement is loaded into a delivery device and several vertebrae are repaired in a single medical procedure. Optionally, one or more cannulae or needles are employed to perform multiple procedures.

Viscous cements according to exemplary embodiments of the invention may be delivered at a desired flow rate through standard orthopedic cannulae by applying sufficient pressure. Exemplary average injection rates may be in the range of 0.01 to 0.5 ml/sec, optionally about 0.05, about 0.075 or 0.1 ml/sec or lesser or intermediate or greater average flow rates. Optionally, the flow rate varies significantly during an injection period (e.g., pulse injections). Optionally, the flow rate is controlled manually or using electronic or mechanical circuitry. In an exemplary embodiment of the invention; medical personnel view the cement as it is being injected (e.g. via fluoroscopy) and adjust a flow rate and/or delivery volume based upon observed results. Optionally; the flow rate is adjusted and/or controlled to allow a medical practitioner to evaluate progress of the procedure based upon medical images (e.g. fluoroscopy) acquired during the procedure. In an exemplary embodiment of the invention, the cement is sufficiently viscous that advances into the body when pressure is applied above a threshold and ceases to advance when pressure is reduced below a threshold. Optionally, the threshold varies with one or more of cement viscosity, cannula diameter and cannula length.

### Comparison of exemplary formulations according to some embodiments of the invention to previously available formulations

Although PMMA has been widely used in preparation of bone cement, previously available PMMA based cements were typically characterized by a persistent liquid state after mixing of components.

In sharp contrast, cements according to some exemplary embodiments of the invention are characterized by essentially no liquid state. Optionally, a direct transition from separate polymer and monomer components to a highly viscous state results from the presence of two or more sub-populations of polymer beads.

As a result of formulations based upon bead sub-populations, a viscosity profile of a cement according to an exemplary embodiment of the invention is significantly different from a viscosity profile of a previously available polymer based cement (e.g. PMMA) with a similar average molecular.

Because the viscosity profile of previously available PMMA cements is typically characterized by a rapid transition from high viscosity to fully hardened, these cements are typically injected into bone in a liquid phase so that they do not harden during injection.

In sharp contrast, exemplary cements according to the invention remain highly viscous during a long working window 114 before they harden. This long working window permits performance of a medical procedure of several minutes duration and imparts the advantages of the high viscosity material to the procedure.

It should be noted that while specific examples are described, it is often the case that the formulation will be varied to achieve particular desired mechanical properties. For example, different diagnoses may suggest different material viscosities which may, in turn lead to adjustment of one or more of MW (average and/or distribution), bead size and bead surface area.

In an exemplary embodiment of the invention, the cement is mixed 112 and reaches high viscosity outside the body. Optionally the materials are mixed under vacuum or ventilated. In this manner, some materials with potentially hazardous by-products can be safely mixed and then used in the body.

In an exemplary embodiment of the invention, the cement is formulated so that its mechanical properties match the bone in which it will be injected/implanted. In an exemplary embodiment of the invention, the cement is formulated to mechanically match healthy or osteoporotic trabecular (cancellous) bone. Optionally, the mechanical properties of the bone are measured during access, for example, based on a resistance to advance or using sensors provided through a cannula or by taking samples, or based on x-ray densitometry measurements. In an exemplary embodiment of the invention, strength of the cement varies as a function of one or more of a size of the high MW sub-population and/or a relationship between bead size and bead MW.

In general, PMMA is stronger and has a higher Young modulus than trabecular bone. For example, healthy Trabecular bone can have a strength of between 1.5-8.0 mega Pascal and a Young modulus of 60-500 mega Pascal. Cortical bone, for example, has strength values of 65-160 mega Pascal and Young modulus of 12-40 giga Pascal. PMMA typically has values about half of Cortical bone (70-120 mega Pascal strength).

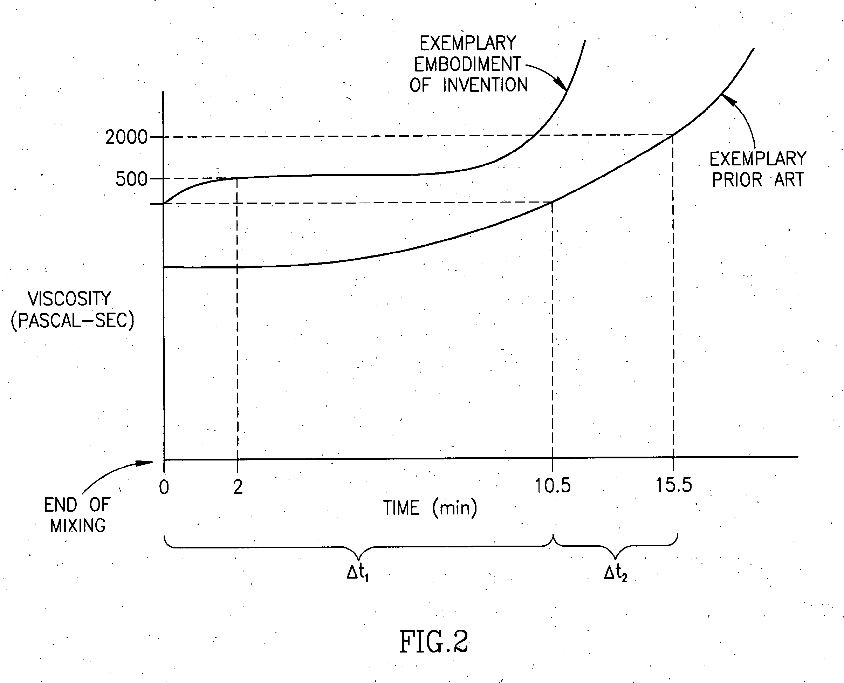

Fig. 2 is a plot of viscosity as a function of time for an exemplary bone cement according to the present invention. The figure is not drawn to scale and is provided to illustrate the principles of exemplary embodiments of the invention. The end of a mixing process is denoted as time 0. Mixing is deemed to end when 95-100% of acrylic polymer beads have been wetted with monomer. The graph illustrates an exemplary bone cement which enters a high viscosity plastic phase upon mixing so that it has substantially no liquid phase.

Fig. 2 illustrates that once a high viscosity is achieved, the viscosity remains relatively stable for 2, optionally 5, optionally 8 minutes or more. In an exemplary embodiment of the invention, this interval of stable viscosity provides a working window 114 (indicated here as Δt₁) for performance of a medical procedure. In an exemplary embodiment of the invention, stable viscosity means that the viscosity of the cement changes by less than 200 Pascal-second during a window of at least 2 minutes optionally at least 4 minutes after mixing is complete. Optionally, the window begins 1, 2, 3, 4 or 5 minutes after mixing begins or lesser or intermediate times. In an exemplary embodiment of the invention, the viscosity of the cement remains below 1500, optionally 2000 Pascal-second for at least 4, optionally at least 6, optionally at least 8, optionally at least 10 minutes or intermediate or greater times from onset of mixing.

For purposes of comparison, the graph illustrates that an exemplary prior art cement reaches a viscosity comparable to that achieved by an exemplary cement according to the invention at time zero at a time of approximately 10.5 minutes post mixing and is completely set by about 15.5 minutes (Δt₂).

A working window 114 during which viscosity is between 400 and 2000 Pascal-second for an exemplary cement according to some embodiments of the invention (Δt₁) is both longer and earlier than a comparable window for an exemplary prior art cement (Δt₂). Optionally, (Δt₁) begins substantially as soon as mixing is complete.

### Exemplary cement formulations

according to various exemplary embodiments of the invention, changes in the ratios between a powdered polymer component and a liquid monomer component can effect the duration of working window 114 and/or a viscosity of the cement during that window. Optionally, these ratios are adjusted to achieve desired results.

In an exemplary embodiment of the invention, the powdered polymer component contains PMMA (69.3% w/w); barium sulfate (30.07% w/w) and benzoyl peroxide (0.54% w/w).

In an exemplary embodiment of the invention, the liquid monomer component contains MMA (98.5% v/v); N, N-dimethyl-p-toluidine (DMPT) (1.5% v/v) and hydroquinone (20 ppm).

In a first exemplary embodiment of the invention, 20 ± 0.3 grams of polymer powder and 9 ± 0.3grams of liquid monomer are combined (weight ratio of ~2.2:1).

In a second exemplary embodiment of the invention, 20 ± 0.3 grams of polymer powder and 8 ± 0.3 grams of liquid are combined (weight ratio of 2.5:1).

Under same weight ratio of second exemplary embodiment (2.5:1), a third exemplary embodiment may include a combination of 22.5 ± 0.3 grams of polymer powder and 9 ± 0.3 grams of liquid.

In general, increasing the weight ratio of polymer to monomer produces a cement which reaches a higher viscosity in less time. However, there is a limit beyond which there is not sufficient monomer to wet all of the polymer beads.

Optionally the powdered polymer component may vary in composition and contain PMMA (67-77%, optionally 67.5-71.5% w/w); Barium sulfate (25-35%; optionally 28-32% w/w) and Benzoyl peroxide (0.4-0.6% w/w) and still behave substantially as the powder component recipe set forth above.

Optionally the liquid monomer component may vary in composition and contain Hydroquinone (1-30 ppm; optionally 20-25 ppm) and still behave substantially as the liquid component recipe set forth above.

### Viscosity measurements over time for exemplary cements

In order to evaluate the viscosity profile of different exemplary batches of cement according to some embodiments of the invention, a bulk of pre-mixed bone cement is placed inside a Stainless Steel injector body. Krause et al. described a method for calculating viscosity in terms of applied force. ("The viscosity of acrylic bone cements", Journal of Biomedical Materials Research, (1982): 16:219-243).

In the experimental apparatus an inner diameter of the injector body is approximately 18 mm. A distal cylindrical outlet has an inner diameter of approximately 3 mm and a length of more than 4 mm. This configuration simulates a connection to standard bone cement delivery cannula/bone access needle. A piston applies force (F), thus causing the bone cement to flow through the outlet. The piston is set to move with constant velocity of approximately 3 mm/min. As a result, piston deflection is indicative of elapsed time.

The experimental procedure serves as a kind of capillary extrusion rheometer. The rheometer measures the pressure difference from an end to end of the capillary tube. The device is made of an 18 mm cylindrical reservoir and a piston. The distal end of the reservoir consist of 4 mm long 3 mm diameter hole. This procedure employs a small diameter needle and high pressure. Assuming steady flow, isothermal conditions and incompressibility of the tested material, the viscous force resisting the motion of the fluid in the capillary is equal to the applied force acting on the piston measured by a load cell and friction. Results are presented as force vs. displacement. As displacement rate was constant and set to 3 mm/min, the shear rate was constant as well. In order to measure the time elapses from test beginning, the displacement rate is divided by 3 (jog speed).

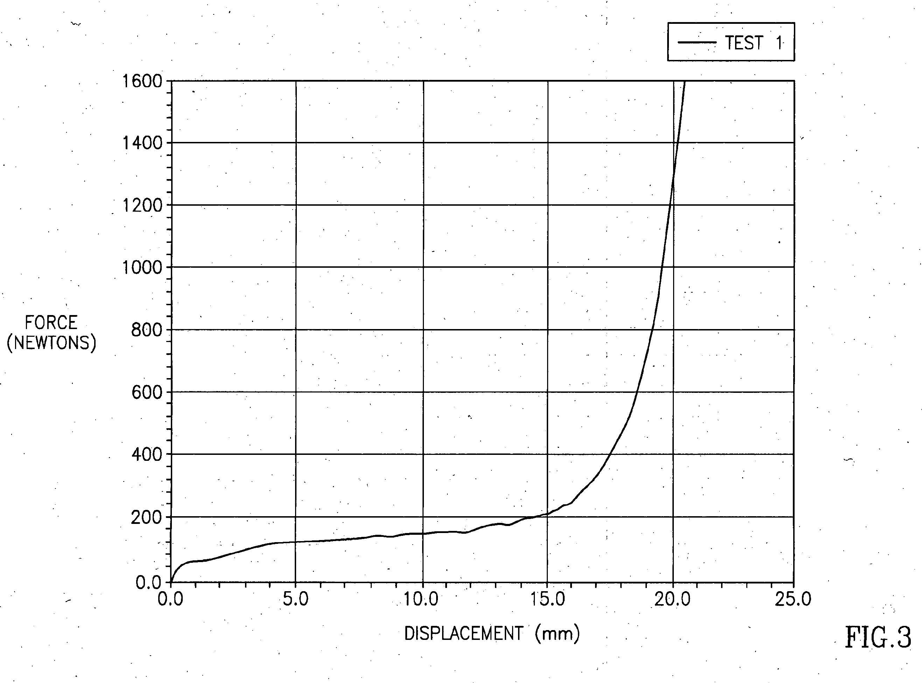

Fig. 3 indicates a viscosity profile of a first exemplary batch of cement according to the invention as force (Newtons) vs. displacement (mm). The cement used in this experiment included a liquid component and a powder component as described above in "Exemplary cement formulations".

In this test (Average temperature: 22.3°C; Relative Humidity: app. 48%) the cement was mixed for 30-60 seconds, then manipulated by hand and placed inside the injector. Force was applied via the piston approximately 150 seconds after end of mixing, and measurements of force and piston deflection were taken.

At a time of 2.5 minutes after mixing (0 mm deflection) the force applied was higher than 30 N.

At a time of 6.5 minutes after mixing (12 mm deflection) the force applied was about 150 N.

At a time of 7.5 minutes after mixing (15 mm deflection) the force applied was higher than 200 N.

At a time of 8.5 minutes after mixing (18 mm deflection) the force applied was higher than 500 N.

At a time of 9.17 minutes after mixing (20 mm deflection) the force applied was higher than 1300 N.

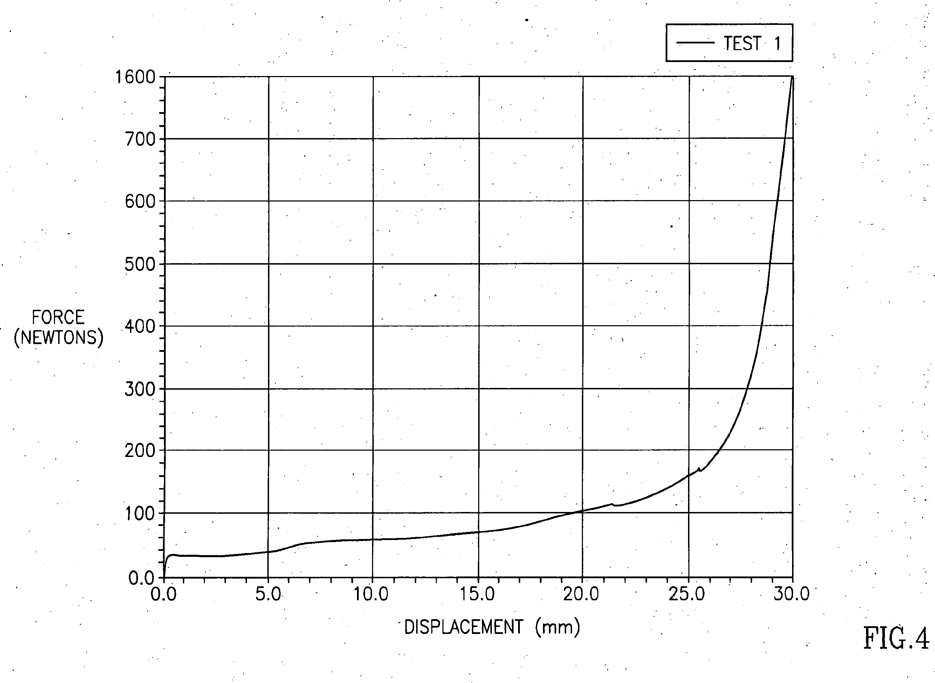

Fig. 4 indicates a viscosity profile of an additional exemplary batch of cement according to the invention as force (Newtons) vs. displacement (mm). The cement in this test was prepared according to the same formula described for the experiment of Fig. 3. In this test (Average 21.1 °C; Relative Humidity: app. 43%) the cement was mixed for approximately 45 seconds, then manipulated by hand and placed inside the injector. Force was applied via piston approximately 150 seconds after end of mixing, and measurements of force and piston deflection were taken. At a time of 2.25 minutes after mixing (0 mm deflection) the force applied was higher than 30 N.

At a time of 8.25 minutes after mixing (18 mm deflection) the force applied was about 90 N.

At a time of 10.3 minutes after mixing (25 mm deflection) the force applied was higher than 150 N.

At a time of 11.4 minutes after mixing (28.5 mm deflection) the force applied was higher than 500 N.

At a time of 12.25 minutes after mixing (30 mm deflection) the force applied was higher than 800 N. Results shown in Figs. 3 and 4 and summarized hereinabove illustrate that exemplary bone cements according to some embodiments the invention achieve high viscosity in 2.25 minutes or less after mixing is completed. Alternatively or additionally, these cements are characterized by short mixing time (i.e. transition to highly viscous plastic phase in 30 to 60 seconds). The exemplary cements provide a "working window" for injection of 4.5 to 6.3 minutes, optionally longer if more pressure is applied and/or ambient temperatures are lower. These times correspond to delivery volumes of 14.9 and 20.8 ml respectively (vertebroplasty of a single vertebra typically requires about 5 ml of cement). These volumes are sufficient for most vertebral repair procedures. These results comply with the desired characteristics described in Fig. 2. Differences between the two experiments may reflect the influence of temperature and humidity on reaction kinetics.

### Molecular weight distribution

The average molecular weight (MW) is skewed by the presence of one or more small sub-population of beads with a molecular weight which is significantly different from a main sub-population of polymer beads.

The presence of even a relatively small sub-population of polymer beads with a MW significantly above the average MW causes the cement to achieve a high viscosity in a short time after wetting of polymer beads with monomer solution. Optionally, increasing a size of the high MW sub-population increases the achieved viscosity. Alternatively or additionally, increasing an average MW of the high MW sub-population increases the achieved viscosity and/or decreases the time to reach high viscosity. Optionally, the one or more small sub-population of beads are provided in a formulation in which, the average molecular weight of PMMA in all beads is optionally 160,000, optionally 180,000, optionally, 250,000, optionally 325,000, optionally 375.000, optionally 400,000, optionally 500,000 Dalton or intermediate or lesser or greater values. In another exemplary embodiment of the invention, the average molecular weight of the acrylic polymer in the beads is in the range of about 150,000 to 170,000, optionally about 160,000 Dalton.

The main sub-population of PMMA beads has a MW of 150,000 Dalton to 500,000 Dalton, optionally about 250,000 Dalton to about 300,000 Dalton, optionally about 275,000 Dalton to about 280,000 Dalton. Optionally, about 90-98% [w/w], optionally about 93% to 98%, optionally about 95% to 97% of the beads belong to the main sub-population.

The second high MW sub-population of PMMA beads has a MW of 600,000 Dalton, to 5,000,000 Dalton, optionally about 3,000,000 Dalton to about 4,000,000 Dalton, Optionally about 3,500,000 Dalton to about 3,900,000 Dalton. Optionally, approximately 0.25% to 5% [w/w], optionally about 1% to 4%, optionally about 2% to 3% of the beads belong to this high MW sub-population. Optionally, this high molecular weight sub-population comprises a styrene co-polymer. In an exemplary embodiment of the invention, a higher molecular weight in this sub-population of beads contributes to a high viscosity within 2, optionally within 1, optionally within 0.5 minutes or less of wetting of polymer beads with monomer solution.

In an exemplary embodiment of the invention, a third low MW sub-population of PMMA beads has a MW in the range of about 1,000 Dalton to about 75,000 Dalton, optionally about 10,000 Dalton to about 15,000 Dalton, optionally about 11,000 Dalton to about 13,000 Dalton. Optionally, approximately 0.5 to 2.0% [w/w], optionally about 1% of the beads belong to this sub-population.

Optionally the MW sub-populations are distinct from one another. This can cause gaps between sub-populations with respect to one or more parameters. In an exemplary embodiment of the invention, the sub-populations are represented as distinct peaks in a chromatographic separation process. Optionally, the peaks are separated by a return to baseline. Depending upon the sensitivity of detection, a background level of noise may be present. Optionally, gaps are measured relative to the noise level.

Optionally the sub-populations abut one another so that no gaps are apparent. In an Exemplary embodiment of the invention, the sub-populations are represented as overlapping peaks in a chromatographic separation process. In this case, there is no return to baseline between the peaks.

### Experimental analysis of an exemplary batch of cement

Sub-populations characterized by an average molecular weight were identified and quantitated using chromatographic techniques known in the art. Exemplary results described herein are based upon GPC analysis. Each peak in the GPC analysis is considered a sub-population. Similar analyses may be conducted using HPLC. Results are summarized in table 1.

**Table I: MW distribution of polymer beads based upon GPC analysis of a bone cement according to the powdered polymer component described in "Exemplary cement formulations" hereinabove.**

| Fraction | % of total | PDI¹ | Mw² | Mn³ |
|---|---|---|---|---|
| 1 | 96.5 | 1.957 | 278,986 | 142,547 |
| 2 | 2.5 | 1.048 | 3,781,414 | 3,608,941 |
| 3 | 1.0 | 1.009 | 12,357 | 12,245 |
| | 100.0 | 2.955 | 373,046 | 126,248 |

| | | | | |
|---|---|---|---|---|
| 1 polydispersity index (PDI), is a measure of the distribution of molecular weights in a given polymer sample and is equal to MW/Mn. 2 MW is the weight average molecular weight in Daltons 3 Mn is the number average molecular weight in Daltons | | | | |

Table I illustrates an exemplary embodiment of the invention with three sub-populations of acrylic polymer beads.

The main sub-population (fraction 1) of PMMA beads has a molecular weight (MW) of 278,986 Dalton. About 96.5% of the beads belong to this sub-population.

A second sub-population (fraction 2) of PMMA beads has MW of 3,781,414 Dalton. Approximately 2.5% of the beads belong to this sub-population.

A third sub-population of PMMA beads (fraction 3) has an MW of 12,357 Dalton. Approximately 1% of the beads belong to this sub-population.

In an exemplary embodiment of the invention, cement comprising these three sub-populations is characterized by a short mixing time and/or achieves a viscosity of 500 to 900 Pascal-second in 0.5 to 3, optionally 0.5 to 1.5 minutes from the beginning of mixing and/or which remains below 2000 Pascal-second for at least 6 to 10 minutes after mixing. A short mixing time followed by a long working window is considered advantageous in orthopedic procedures where operating room availability and medical staff are at a premium.

### Size Distribution

In an exemplary embodiment of the invention, the bone cement is characterized by beads with a size distribution including at least two sub-populations of polymer beads.

In an exemplary embodiment of the invention, polymer bead diameter is in the range of 10-250 microns, with a mean value of approximately 25, 30, 40, 50, 60 microns, or a lower or a higher or an intermediate diameter. In an exemplary embodiment of the invention, sub-populations of beads are defined by their size.

Optionally, a main sub-population of polymer (e.g. PMMA) beads is characterized by a diameter of about 20 to about 150, optionally about 25 to about 35, optionally an average of about 30 microns. Beads in this main sub-population are optionally far smaller than the smallest beads employed by Hernandez et al. (2005; as cited above). Presence of small beads can contribute to a rapid increase in viscosity after wetting with monomer.

Optionally a second sub-population of large polymer beads is characterized by a diameter of about 150 microns or more. Presence of large beads can slow down the polymerization reaction and prevent hardening, contributing to a long working window.

Optionally, the remaining beads are characterized by a very small average diameter, for example less than 20, optionally less than 15, optionally about 10 microns or less. Presence of very small beads can facilitate rapid wetting with monomer liquid during mixing and contribute to a fast transition to a viscous state with substantially no liquid phase.

Microscopic analysis indicates that the beads are typically spherical or spheroid.

Hernandez et al. (2005; as cited above) examined the possibility of adjusting the average polymer bead size by combining two types of beads with average sizes of 118.4 µ (Colacry) and 69.7µ (Plexigum) together in different ratios. However, Hernandez's goal was a formulation which is "liquid enough to be injected". All formulations described by Hernandez are characterized by an increase in viscosity from 500 Pascal-sec to 2000 Pascal-sec in about two minutes or less (corresponds to window 114). Hernandez does not hint or suggest that there is any necessity or advantage to increasing the size of this window.

Microscopic analysis also indicated that the barium sulfate particles are present as elongate amorphous masses with a length of approximately 1 micron. In some cases aggregates of up to 70 microns in size were observed. In some cases, barium sulfate particles and polymer beads aggregated together. Optionally, aggregates of Barium sulfate and polymer beads can delay wetting of polymer beads by monomer.

In an exemplary embodiment of the invention, MMA solvates and/or encapsulates the PMMA polymer beads and the viscosity of the initial mixture is high due to the solvation and/or friction between the beads. As the beads dissolve viscosity remains high due to polymerization which increases the average polymer MW.

### Size and MW are independent variables

In an exemplary embodiment of the invention, size based and MW based sub-populations are determined independently. For example, MW may be determined chromatographically and size may be determined by microscopic analysis. As a result, beads classed in a single size sub-population may be classed in two or more MW sub-populations and/or beads classed in a single MW sub-population may be classed in two or more size sub-populations.

### Mechanical viscosity increasing agents

In an exemplary embodiment of the invention, the cement includes particles characterized by a large surface which do not participate in the polymerization reaction. The large surface area particles can impart added viscosity to the cement mixture independent of polymerization. Optionally, the added viscosity comes from friction of particles against one another in the cement.

Examples of materials which do not participate in the polymerization reaction but increase viscosity include, but are not limited to Zirconium, hardened acrylic polymer, barium sulfate and bone.

Optionally, materials which do not participate in the polymerization reaction but increase viscosity can at least partially substitute for high MW polymers in influencing a viscosity profile.

### Desired Polymerization Reaction Kinetics

In an exemplary embodiment of the invention, mixture of polymer and monomer produces a high viscosity mixture with substantially no intervening liquid phase within 180, optionally within 120, optionally within 100, optionally within 60, optionally within 30, optionally within 15 seconds or greater or intermediate times from onset of mixing.

In an exemplary embodiment of the invention, once a high viscosity is achieved, the viscosity remains stable for 5 minutes, optionally 8 minutes, optionally 10 minutes or lesser or intermediate or greater times. Optionally, stable viscosity indicates a change of 10% or less in two minutes and/or a change of 20% or less in 8 minutes. The time during which viscosity is stable provides a working window for performance of a medical procedure.

These desired reaction kinetics can be achieved by adjusting one or more of average polymer MW, polymer MW distribution, polymer to monomer ratio and polymer bead size and/or size distribution.

### General considerations

In an exemplary embodiment of the invention, a powdered polymer component and a liquid monomer component are provided as a kit. Optionally, the kit includes instructions for use. Optionally, the instructions for use specify different proportions of powder and liquid for different desired polymerization reaction kinetics.

In an exemplary embodiment of the invention, a bone cement kit including at least two, optionally three or more separately packaged sub-populations of beads and a monomer liquid is provided. Optionally, the kit includes a table which provides formulations based on combinations of different amounts of bead sub-populations and monomer to achieve desired properties.

It is common practice in formulation of acrylic polymer cements to include an initiator (e.g. benzoyl peroxide; BPO) in the powdered polymer component and/or a chemical activator (e.g. DMPT) into the liquid monomer component. These components can be added to formulations according to exemplary embodiments of the invention without detracting from the desired properties of the cement.

Optionally, an easily oxidized molecule (e.g. hydroquinone) is added to the liquid component to prevent spontaneous polymerization during storage (stabilizer). The hydroquinone can be oxidized during storage.

Optionally, cement may be rendered radio-opaque, for example by adding a radio-opaque material such as barium sulfate and/or zirconium compounds and/or bone (e.g. chips or powder) to the powder and/or liquid component.

While the above description has focussed on the spine, other tissue can be treated as well, for example, compacted tibia plate and other bones with compression fractures and for fixation of implants, for example, hip implants or other bone implants that loosened, or during implantation. Optionally, for tightening an existing implant, a small hole is drilled to a location where there is a void in the bone and material is extruded into the void.

It should be noted that while use of the disclosed material as bone cement is described, non-bone tissue may optionally be treated. For example, cartilage or soft tissue in need of tightening may be injected with a high viscosity polymeric mixture. Optionally, the delivered material includes an encapsulated pharmaceutical and is used as a matrix to slowly release the pharmaceutical over time. Optionally, this is used as a means to provide anti-arthritis drugs to a joint, by forming a void and implanting an eluting material near the joint.

Optionally, at least some of the beads include styrene. In an exemplary embodiment of the invention, styrene is added to MMA beads in a volumetric ratio of 5-25%. Optionally, addition of styrene increases creep resistance. According to various embodiments of the invention, a bone cement according to the invention is injected into a bone void as a preventive therapy and/or as a treatment for a fracture, deformity, deficiency or other abnormality. Optionally, the bone is a vertebral body and/or a long bone. In an exemplary embodiment of the invention, the cement is inserted into the medullary canal of a long bone. Optionally, the cement is moulded into a rod prior to or during placement into the bone. In an exemplary embodiment of the invention, the rod serves as an intra-medullary nail.

### Exemplary Characterization Tools

Molecular weight and polydispersity can be analysed, for example by Gel permeation chromatography(GPC) system (e.g. Waters 1515 isocratic HPLC pump with a Waters 2410 refractive-index detector and a Rheodyne (Coatati, CA) injection valve with a 20- µL loop (Waters Ma)). Elution of samples with CHC13 through a linear Ultrastyragel column (Waters; 500-A pore size) at a flow rate of 1 ml/min provides satisfactory results.

It will be appreciated that various tradeoffs may be desirable, for example, between available injection force, viscosity, degree of resistance and forces that can be withstood (e.g. by bone or injection tools). In addition, a multiplicity of various features, both of method and of cement formulation have been described. It should be appreciated that different features may be combined in different ways. In particular, not all the features shown above in a particular embodiment are necessary in every similar exemplary embodiment of the invention. Further, combinations of the above features are also considered to be within the scope of some exemplary embodiments of the invention. In addition, some of the features of the invention described herein may be adapted for use with prior art devices, in accordance with other exemplary embodiments of the invention.

Section headers are provided only to assist in navigating the application and should not be construed as necessarily limiting the contents described in a certain section, to that section.

Measurements are provided to serve only as exemplary measurements for particular cases, the exact measurements applied will vary depending on the application. When used in the following claims, the terms "comprises", "comprising", "includes", "including" or the like means "including but not limited to".

## Claims

1. A bone cement comprising an acrylic polymer mixture, the cement achieving a viscosity of at least 500 Pa.s within 180 seconds following initiation of mixing of a polymerisable monomer component and a powdered polymer component with which a reaction initiator is included, and the cement having sufficient biocompatibility to permit *in vivo* use, in which the polymer component comprises (i) a main sub-population of PMMA beads having a molecular weight (MW) in a range of between 150,000 Dalton and 500,000 Dalton, and (ii) a high molecular weight sub-population of PMMA beads having a molecular weight in a range between 600,000 Dalton to 5,000,000 Dalton.

2. A bone cement according to claim 1, wherein the PMMA is provided as a PMMA/styrene copolymer.

3. A bone cement according to any of the preceding claims, wherein the mixture includes barium sulphate.

4. A bone cement according to claim 1, wherein the main sub-population of beads has an average molecular weight of from 150,000 Dalton to 300,000 Dalton.

5. A bone cement according to claim 4, wherein the main sub-population of beads includes 90-98% (w/w) of the beads.

6. A bone cement according to claim 1, wherein the high molecular weight sub-population of beads has an average molecular weight of at least 3,000,000 Dalton.

7. A bone cement according to claim 1, wherein the high molecular weight sub-population of beads includes 2 to 3% (w/w) of the beads.

8. A bone cement according to claim 1, which includes a third low molecular weight sub-population of PMMA beads having an average molecular weight of less than 15,000 Dalton.

9. A bone cement according to claim 8, wherein the third low molecular weight sub-population of PMMA beads includes 0.75 to 1.5 % (w/w) of the beads.

10. A bone cement according to claim 1, wherein at least one of the bead sub-population which has an average diameter is further divided into at least two sub-sub-populations, each sub-sub-population having an average molecular weight.

11. A bone cement according to claim 1, wherein the polymer component is provided as a population of beads divided into at least three sub-populations, each sub-population having an average bead diameter.

12. A bone cement according to any of the preceding claims, further comprising processed bone and/or synthetic bone.

13. A bone cement according to any of the preceding claims, in which the cement achieves a viscosity of at least 500 Pa.s when 100% of a polymer component is wetted by a monomer component.

14. A bone cement according to any of the preceding claims, which achieves a viscosity of at least 800 Pa.s within 180 seconds following initiation of mixing of the monomer component and the polymer component.

15. A bone cement according to any of the preceding claims, which achieves a viscosity is at least 1500 Pa.s within 180 seconds following initiation of mixing of the monomer component and the polymer component.

16. A bone cement according to any of the preceding claims, which achieves the viscosity of at least 500 Pa.s within two minutes following initiation of mixing of the monomer component and the polymer component.

17. A bone cement according to any of the preceding claims, which achieves the viscosity of at least 500 Pa.s within one minute following initiation of mixing of the monomer component and the polymer component.

18. A bone cement according to any of the preceding claims, which achieves the viscosity of at least 500 Pa.s within 45 seconds following initiation of mixing of the monomer component and the polymer component.

19. A bone cement according to any of the preceding claims, in which the step of mixing the polymer component and the monomer component produces a mixture which attains a viscosity greater than 200 Pa.s within 1 minute from onset of mixing and remains below 2000 Pa.s until at least 6 minutes from onset of mixing.

20. A bone cement according to claim 19, wherein the polymer component comprises an acrylic polymer.

21. A bone cement according to any of the preceding claims, in which the polymer component comprises:
(a) 60 to 80% polymer beads comprising a main sub-population having a molecular weight of 150,000 Dalton to 300,000 Dalton and a high molecular weight sub-population having a molecular weight of 3,000,000 Dalton to 4,000,000 Dalton; and
(b) 20 to 40% of a material which is non-transparent with respect to X-ray.

22. A bone cement according to claim 21, wherein the polymer beads comprise a third sub-population having a molecular weight of 10,000 Dalton to 15,000 Dalton.

## Patentansprüche

1. Knochenzement, der eine Acrylpolymermischung umfasst, wobei der Zement eine Viskosität von wenigstens 500 Pa.s innerhalb von 180 Sekunden im Anschluss an den Beginn des Mischens einer polymerisierbaren Monomerkomponente und einer pulverisierten Polymerkomponente, mit der ein Reaktionsinitiator eingeschlossen ist, erreicht und der Zement eine ausreichende Biokompatibilität aufweist, um *in*-*vivo-*Gebrauch zu erlauben, wobei die Polymerkomponente (i) eine Hauptunterpopulation von PMMA-Perlen mit einem Molekulargewicht (MG) in einem Bereich von zwischen 150.000 Dalton und 500.000 Dalton und (ii) eine Unterpopulation von PMMA-Perlen mit hohem Molekulargewicht mit einem Molekulargewicht in einem Bereich zwischen 600.000 Dalton bis 5.000.000 Dalton umfasst.

2. Knochenzement nach Anspruch 1, wobei das PMMA als ein PMMA/StyrolCopolymer vorgelegt ist.

3. Knochenzement nach einem der vorangehenden Ansprüche, wobei die Mischung Bariumsulfat einschließt.

4. Knochenzement nach Anspruch 1, wobei die Hauptunterpopulation von Perlen ein durchschnittliches Molekulargewicht von 150.000 Dalton bis 300.000 Dalton aufweist.

5. Knochenzement nach Anspruch 4, wobei die Hauptunterpopulation von Perlen 90-98 Gew.-% der Perlen einschließt.

6. Knochenzement nach Anspruch 1, wobei die Unterpopulation von Perlen mit hohem Molekulargewicht ein durchschnittliches Molekulargewicht von wenigstens 3.000.000 Dalton aufweist.

7. Knochenzement nach Anspruch 1, wobei die Unterpopulation von Perlen mit hohem Molekulargewicht 2 bis 3 Gew.-% der Perlen einschließt.

8. Knochenzement nach Anspruch 1, der eine dritte Unterpopulation von PMMA-Perlen mit niedrigem Molekulargewicht mit einem durchschnittlichen Molekulargewicht von weniger als 15.000 Dalton einschließt.

9. Knochenzement nach Anspruch 8, wobei die dritte Unterpopulation von PMMA-Perlen mit niedrigem Molekulargewicht 0,75 bis 1,5 Gew.-% der Perlen einschließt.

10. Knochenzement nach Anspruch 1, wobei wenigstens eine der Perlen-Unterpopulation, die einen durchschnittlichen Durchmesser aufweist, weiter in wenigstens zwei Unterunterpopulationen aufgeteilt ist, wobei jede Unterunterpopulation ein durchschnittliches Molekulargewicht aufweist.

11. Knochenzement nach Anspruch 1, wobei die Polymerkomponente als eine Population von Perlen vorgelegt ist, die in wenigstens drei Unterpopulationen aufgeteilt ist, wobei jede Unterpopulation einen durchschnittlichen Perlendurchmesser aufweist.

12. Knochenzement nach einem der vorangehenden Ansprüche, der weiter erarbeiteten Knochen und/oder synthetischen Knochen umfasst.

13. Knochenzement nach einem der vorangehenden Ansprüche, in dem der Zement eine Viskosität von wenigstens 500 Pa.s erreicht, wenn 100% einer Polymerkomponente mit einer Monomerkomponente benetzt sind.

14. Knochenzement nach einem der vorangehenden Ansprüche, der eine Viskosität von wenigstens 800 Pa.s innerhalb von 180 Sekunden im Anschluss an den Beginn des Mischens der Monomerkomponente und der Polymerkomponente erreicht.

15. Knochenzement nach einem der vorangehenden Ansprüche, der eine Viskosität von wenigstens 1500 Pa.s innerhalb von 180 Sekunden im Anschluss an den Beginn des Mischens der Monomerkomponente und der Polymerkomponente erreicht.

16. Knochenzement nach einem der vorangehenden Ansprüche, der die Viskosität von wenigstens 500 Pa.s innerhalb von zwei Minuten im Anschluss an den Beginn des Mischens der Monomerkomponente und der Polymerkomponente erreicht.

17. Knochenzement nach einem der vorangehenden Ansprüche, der die Viskosität von wenigstens 500 Pa.s innerhalb einer Minute im Anschluss an den Beginn des Mischens der Monomerkomponente und der Polymerkomponente erreicht.

18. Knochenzement nach einem der vorangehenden Ansprüche, der die Viskosität von wenigstens 500 Pa.s innerhalb von 45 Sekunden im Anschluss an den Beginn des Mischens der Monomerkomponente und der Polymerkomponente erreicht.

19. Knochenzement nach einem der vorangehenden Ansprüche, in dem der Schritt des Mischens der Polymerkomponente und der Monomerkomponente eine Mischung erzeugt, die eine Viskosität von mehr als 200 Pa.s innerhalb von 1 Minute nach dem Einsetzen des Mischens erreicht und bis wenigstens 6 Minuten nach dem Einsetzen des Mischens unterhalb 2000 Ps.s bleibt.

20. Knochenzement nach Anspruch 19, wobei die Polymerkomponente ein Acrylpolymer umfasst.

21. Knochenzement nach einem der vorangehenden Ansprüche, in dem die Polymerkomponente:
(a) 60 bis 80% Polymerperlen, die eine Hauptunterpopulation mit einem Molekulargewicht von 150.000 Dalton bis 300.000 Dalton und eine Unterpopulation mit einem hohen Molekulargewicht mit einem Molekulargewicht von 3.000.000 Dalton bis 4.000.000 Dalton umfasst; und
(b) 20 bis 40% eines Materials, das in Bezug auf Röntgenstrahlen nicht-durchlässig ist,
umfasst.

22. Knochenzement nach Anspruch 21, wobei die Polymerperlen eine dritte Unterpopulation mit einem Molekulargewicht von 10.000 Dalton bis 15.000 Dalton umfassen.

## Revendications

1. Ciment osseux comprenant un mélange de polymères acryliques, le ciment acquérant une viscosité d'au moins 500 Pa.s dans les 180 secondes qui suivent l'initiation du mélange d'un monomère polymérisable et d'un composant polymère sous forme de poudre dans lequel est inclus un initiateur réactionnel, et le ciment ayant une biocompatibilité suffisante pour permettre son utilisation *in vivo,* dans lequel le composant polymère comprend (i) une sous-population principale de billes de PMMA ayant un poids moléculaire (PM) compris dans la plage allant de 150 000 Daltons à 500 000 Daltons, et (ii) une sous-population de poids moléculaire élevé de billes de PMMA ayant un poids moléculaire compris dans la plage allant de 600 000 Daltons à 5 000 000 Daltons.

2. Ciment osseux selon la revendication 1, dans lequel le PMMA est utilisé sous la forme d'un copolymère de PMMA/styrène.

3. Ciment osseux selon l'une quelconque des revendications précédentes, dans lequel le mélange comprend du sulfate de baryum.

4. Ciment osseux selon la revendication 1, dans lequel la sous-population principale de billes a un poids moléculaire moyen allant de 150 000 Daltons à 300 000 Daltons.

5. Ciment osseux selon la revendication 4, dans lequel la sous-population principale de billes comprend de 90 à 98 % (m/m) de billes.

6. Ciment osseux selon la revendication 1, dans lequel la sous-population de billes de poids moléculaire élevé a un poids moléculaire moyen d'au moins 3 000 000 Daltons.

7. Ciment osseux selon la revendication 1, dans lequel la sous-population de billes de poids moléculaire élevé comprend de 2 à 3 % (m/m) de billes.

8. Ciment osseux selon la revendication 1, qui comprend une troisième sous-population de billes de PMMA de faible poids moléculaire ayant un poids moléculaire moyen inférieur à 15 000 Daltons.

9. Ciment osseux selon la revendication 8, dans lequel la troisième sous-population de billes de PMMA de faible poids moléculaire comprend de 0,75 à 1,5 % (m/m) de billes.

10. Ciment osseux selon la revendication 1, dans lequel au moins l'une des sous-populations de billes qui a un diamètre moyen est davantage divisée au moins en deux sous-sous-populations, chaque sous-sous-population ayant un poids moléculaire moyen.

11. Ciment osseux selon la revendication 1, dans lequel le composant polymère est utilisé sous la forme d'une population de billes divisée en au moins trois sous-populations, chaque sous-population ayant un diamètre de billes moyen.

12. Ciment osseux selon l'une quelconque des revendications précédentes, comprenant en outre un os synthétique et/ou un os traité.

13. Ciment osseux selon l'une quelconque des revendications précédentes, dans lequel le ciment acquiert une viscosité d'au moins 500 Pa.s lorsque 100 % d'un composant polymère sont mouillés par un composant monomère.

14. Ciment osseux selon l'une quelconque des revendications précédentes, qui acquiert une viscosité d'au moins 800 Pa.s dans les 180 secondes qui suivent l'initiation du mélange du composant monomère et du composant polymère.

15. Ciment osseux selon l'une quelconque des revendications précédentes, qui acquiert une viscosité d'au moins 1500 Pa.s dans les 180 secondes qui suivent l'initiation du mélange du composant monomère et du composant polymère.

16. Ciment osseux selon l'une quelconque des revendications précédentes, qui acquiert une viscosité d'au moins 500 Pa.s dans les deux minutes qui suivent l'initiation du mélange du composant monomère et du composant polymère.

17. Ciment osseux selon l'une quelconque des revendications précédentes, qui acquiert une viscosité d'au moins 500 Pa.s dans la minute qui suit l'initiation du mélange du composant monomère et du composant polymère.

18. Ciment osseux selon l'une quelconque des revendications précédentes, qui acquiert une viscosité d'au moins 500 Pa.s dans les 45 secondes qui suivent l'initiation du mélange du composant monomère et du composant polymère.

19. Ciment osseux selon l'une quelconque des revendications précédentes, dans lequel l'étape de mélange du composant polymère et du composant monomère produit un mélange qui atteint une viscosité supérieure à 200 Pa.s dans la minute qui suit l'initiation du mélange et reste inférieure à 2000 Pa.s jusqu'à au moins 6 minutes après l'initiation du mélange.

20. Ciment osseux selon la revendication 19, dans lequel le composant polymère comprend un polymère acrylique.

21. Ciment osseux selon l'une quelconque des revendications précédentes, dans lequel le composant polymère comprend :
(a) de 60 à 80 % de billes de polymères comprenant une sous-population principale ayant un poids moléculaire de 150 000 Daltons à 300 000 Daltons et une sous-population de poids moléculaire élevé ayant un poids moléculaire de 3 000 000 Daltons à 4 000 000 Daltons ; et
(b) de 20 à 40 % d'une substance qui n'est pas transparente vis-à-vis des rayons X.

22. Ciment osseux selon la revendication 21, dans lequel les billes de polymère comprennent une troisième sous-population ayant un poids moléculaire de 10 000 Daltons à 15 000 Daltons.
